# EUROPEAN PATENT SPECIFICATION

(11) **EP 2 076 247 B1**
(45) Date of publication and mention of the grant of the patent: **23.11.2016**
(21) Application number: 07867189.8
(22) Date of filing: 02.10.2007
(51) Int. Cl.: A61K 9/14, A61K 9/16, A61P 23/02

(54) **A SELF-GELLING TUNABLE DRUG DELIVERY SYSTEM**
SELBST-GELIERENDES EINSTELLBARES ARZNEIABGABESYSTEM
SYSTÈME D'ADMINISTRATION DE MÉDICAMENT ACCORDABLE AUTOGÉLIFIANT

(30) Priority: 27.10.2006 US 588612
(43) Date of publication of application: 08.07.2009
(73) Proprietor: ETHICON, INC., Somerville, NJ 08876 (US); Schachter, Deborah M., Edison NJ 08817 (US); Zhou, Yue, Horseheads NY 14845 (US)
(72) Inventor: SCHACHTER, Deborah M., Edison, NJ 08817 (US); ZHOU, Yue, Horseheads, NY 14845 (US)
(74) Representative: Kirsch, Susan Edith
(86) International application number: PCT/US2007/021139
(87) International publication number: WO 2008/060365

(56) References cited:
- EP-A1- 1 595 534
- WO-A1-2006/053836
- US-A1- 2003 203 030
- US-A1- 2006 188 583

## Description

### FIELD OF THE INVENTION

The present invention relates to drug delivery systems. Specifically, this invention relates to a self-gelling, tunable drug delivery system having a hydrophobic matrix and a hydrophilic matrix.

### BACKGROUND OF THE INVENTION

The controlled release of drugs from polymer matrices is widely known. There are controlled release systems that are meant to release large amounts of drug in a short period of time. These are typically oral delivery systems and are designed to release an active agent from a polymer matrix over a period of approximately 12 hours. These systems are typically designed to deliver drugs by diffusion. A hydrophobic coating may be employed to reduce the rate of diffusion from the polymer matrix. Generally, it is not desirable for polymers used in oral controlled release systems to erode too slowly. If the polymer matrix erodes too slowly, the formulation will have passed through the patient's digestive system before the majority of the drug is released.

Alternatively, there are controlled release systems that are designed for drug release on a significantly longer timescale. These controlled release systems are typically implantable in the patient. The release of a drug or drugs from implantable controlled release systems is typically influenced by both diffusion and degradation of the polymer matrix. The literature is replete with examples investigating those polymer matrix parameters that most affect the release rate of the drug. In general, the polymer matrix parameters that influence the drug release rate include chemical structure of the polymer, initial molecular weight of the polymer, excipients, crystallinity of the polymer, and the like.

However, even when these parameters are tightly controlled it is not possible to achieve the desired level of control over the amount of drug released on each day. The initial release of the drug, referred to as the burst, arises from the drug that is on or near the surface of the polymer matrix, and this is generally followed by release as the result of erosion of the polymer. Drug release that is diffusion-related and drug release that is erosion-related occur simultaneously, thereby increasing the complexity of the system.

A considerable amount of work has gone into mathematical modeling to predict drug release rates from polymers. Early models, such as those described in Higuchi, T., Rate of Release of Medicaments from Ointment Bases Containing Drugs in Suspension, J Pharm Sci 50 (10) 1960 874-875, only took diffusion into account. Models have been improved considerably since then by including a factor for polymer erosion (see the following: Faisant, J. et al., PLGA-based Microparticle: Elucidation of Mechanisms and A New Simple Mathematical Model Quantifying Drug Release, European Journal of Pharmaceutical Science 15 (2002) 355-366; Zang, H. et al., Simulation of Drug Release From Biodegradable Polymeric Microspheres with Bulk and Surface Erosions. J Pharm Sci. 92 (10) 2003; Chandrashekar, R. et al., Modeling Small Molecule Release From PLG Microspheres: Effects of Polymer Degradation and Nonuniform Drug Distribution. Journal of Controlled Release 103 (2005) 149 - 158). Release rates are still unpredictable, since the chemical composition of the polymer matrix changes as the polymer matrix erodes. For example, during degradation of a polyester matrix, the ester groups in the polymer chain may react with water to form carboxylic acid groups. The carboxylic acid groups can then interact with functional groups on the drug dispersed within polymer matrix. These interactions can impact diffusion rates considerably (Frank, A. et al., Controlled Release from Bioerodible Polymers: Effect of Drug Type and Polymer Composition. Journal of Controlled Release 102 (2005) 333 - 344).

WO-A-2006053836 describes a hydrogel matrix that may contain two or more biodegradable microcarriers dispersed therein for release of two or more different active agents.

EP-A-1595534 likewise describes aqueous gel compositions comprising two different kinds of microparticles. The microparticles appear to be formed of hydrophilic gel-forming polymers having different ionic charges

US-A-20060188583 describes hydrogels containing dispersed biodegradable microspheres containing therapeutic agents. The hydrogel itself may also contain a therapeutic agent.

A need still remains for a novel polymer matrix controlled release system with tunable release rates.

### SUMMARY OF THE INVENTION

The present invention provides a self-gelling, tunable drug delivery system comprising: a hydrophilic matrix, which is comprised of a hydrophilic polymer that swells upon contact with a hydrating agent thereby creating a hydrogel, and a first drug, wherein said hydrophilic matrix is in the form of pellets, ribbons, films, tapes, strips, tablets or granules, and a hydrophobic matrix, which is comprised of a hydrophobic polymer and a second drug, wherein said hydrophobic matrix is in the form of pellets, ribbons, films, tapes, strips, tablets or granules, wherein the hydrophilic and hydrophobic matrices may be combined, hydrated, and then placed in situ, or combined, placed in situ, and then hydrated.

The hydrophilic matrix swells and forms a hydrogel upon contact with a hydrating agent and suspends the hydrophobic matrix in place. Drug release from the hydrogel is rapid, while release from the hydrophobic matrix is dependent on polymer degradation rate.

The self-gelling tunable drug delivery system is useful for treating a variety of medical conditions and indications, including the treatment of pain, infection, and inflammation at the site of injury.

### DETAILED DESCRIPTION OF THE INVENTION

The self-gelling, tunable drug delivery system of the present invention is comprised of a hydrophilic matrix and a hydrophobic matrix as defined in claim 1.

The hydrophilic matrix is comprised of a hydrophilic polymer and a first drug. The hydrophilic polymer is biocompatible and preferably biodegradable. The hydrophilic polymer swells and forms a hydrogel when in contact with a hydrating agent, such as water, phosphate buffered saline, or physiological medium, such as blood. Suitable hydrophilic polymers include, but are not limited to, hydroxyethylcellulose, hydroxypropylmethylcellulose, hydroxymethylcellulose, hydroxypropylcellulose, carboxymethylcellulose, hyaluronic acids, salts hyaluronic acids, such as sodium hyaluronate; alginates, polyvinylpyrrolidone, polyethylene oxide, polysccarrides, chitins, chitosan, gelatin, polyacrylic acid and derivatives, gums (i.e. guar), and polymers derived from starch. In one embodiment, the hydrophilic polymer is a high molecular weight sodium hyaluronate. The hydrophilic polymer is biocompatible and preferably biodegradable.

The hydrophilic matrix is prepared by mixing the first drug with the hydrophilic polymer. A therapeutically effective amount of the first drug is utilized. In one embodiment, the drug loading is about 0.01 to about 55 percent by weight. In another embodiment, the drug loading is about 10 to about 30 percent by weight. A wet granulation of this mixture is prepared using a wetting agent. Wetting agents may include, but are not limited to, water, alcohol, and water/alcohol mixtures. In one embodiment, the wet granulation is placed in the hopper of a single screw extruder. The extrudate is cut or shaped into the desired form, for example, pellets. Other forms of the hydrophilic matrix, depending upon the application include pellets, ribbons, films, tapes, strips, tablets, and granules. Mild processing conditions for the hydrophilic matrix, such as single screw extrusion, are ideal for the incorporation of sensitive drugs, such as proteins.

The hydrophobic matrix is comprised of a hydrophobic polymer and a therapeutically effective amount of a second drug. The hydrophobic polymer is biocompatible and biodegradable. Biodegradable polymers readily break down into small segments when exposed to moist body tissue or physiological enzymes. The segments are either absorbed by the body or passed by the body. More particularly, the biodegraded segments do not elicit permanent chronic foreign body reaction, because they are absorbed by the body or passed from the body such that no permanent trace or residual amount of the segment is retained by the body. In one embodiment, the hydrophobic polymer is melt processable at low temperatures, such as temperatures less than 100°C, allowing processing of certain drugs without degrading or denaturing the drug. In a preferred embodiment, the hydrophobic polymer degrades in about 1 week to about 3 weeks.

In one embodiment, the hydrophobic polymer is an aliphatic polyester. Aliphatic polyesters include, but are not limited to homopolymers, copolymers, and terpolymers of lactide (which includes lactic acid, d-, 1- and meso lactide), glycolide (including glycolic acid), epsilon-caprolactone, p-dioxanone (1,4- dioxan-2-one), and trimethylene carbonate (1, 3-dioxan-2-one). Copolymers and terpolymers include statistically random, block, segmented, and graft polymers. In another embodiment, the hydrophobic polymer is 50/50 mol/mol percent poly(d,1-lactide-co-glycolide). One of skill in the art will be able to identify homopolymers, copolymers, and terpolymers of aliphatic polyesters having a melt processing temperature less than 100°C and having a degradation time of about 1 to about 3 weeks.

The hydrophobic matrix may be prepared by compounding and extruding. In this embodiment, the compounder and extruder are heated to the appropriate temperature for melt processing the hydrophobic polymer, for example a temperature less than 100°C. The second drug and the polymer are weighed out separately. In one embodiment, the drug loading is about 0.01 to about 35 percent by weight. In another embodiment the drug loading is about 10 to about 25 percent by weight. A small fraction of the polymer is left aside, but the remainder is mixed together with the drug powder and placed into heated twin screw extruder. The remaining polymer fraction is then added to the extruder. The mixture is allowed to mix until uniform and then allowed to extrude out. Extrudate is cut or shaped the desired form, for example, into pellets. The form of the hydrophobic matrix may vary depending upon the application and includes pellets, ribbons, films, tapes, strips and tablets.
The extrusion of the hydrophobic matrix at temperatures of less than approximately 100°C allows for the incorporation drugs that are sensitive to high temperatures and thermal degradation without compromising the drug.

The self-gelling tunable, drug delivery system is prepared by combining sufficiently effective amount of the hydrophilic matrix with a sufficiently effective amount of the hydrophobic matrix in a conventional manner (for example, by mixing) and contacting the combination with a sufficiently effective amount of a hydrating agent to created a hydrogel. The hydrophilic matrix swells upon contact with a hydrating agent. Hydrating agents include, but are not limited to water, phosphate buffered saline, or physiological medium, such as blood, forming a hydrogel and the hydrophobic matrix is suspended in place. The hydrophilic and hydrophobic matrices may be combined, hydrated, and then placed in situ, or combined, placed in situ, and then hydrated.

A number of drugs may be used as the first drug and the second drug in the self-gelling, tunable drug delivery system. Choices for the first drug and the second drug include, but are not limited to, anti-infectives, such as anti-bacterials and antibiotics; analgesics, such as nonopioid analgesics, opioid analgesics, nonopioid/opioid analgesic combinations, COX-2 inhibitors, and anti-inflammatory agents, such as nonsteriodal anti-inflammatory agents; anesthetics, such as local anesthetics; immunosupressives, steroids, statins, alpha-2-agonists, VR1 -agonists, proton pump inhibitors, collagen peptides, parathyroid hormone, bone morphogenic proteins, p38 kinase inhibitors and combinations thereof.

In another embodiment, the first drug and the second drug is selected from a pain medication including but not limited to ibuprofen, oxycodone, morphine, fentanyl, hydrocodone, naproxyphene, codeine, acetaminophen with codeine, acetaminophen, benzocaine, lidocaine, procaine, bupivacaine, ropivacaine, mepivacaine, chloroprocaine, tetracaine, cocaine, etidocaine, prilocaine, procaine, clonidine, xylazine, medetomidine, dexmedetomidine, VR1 antagonists and combinations thereof. In another embodiment, the drug is bupivacaine or lidocaine.

In one embodiment, the first drug and the second drug are the same drug. In another embodiment, the first drug and the second drug are different. For example, a protein can be the first drug in the hydrophilic matrix while an NSAID can be the second drug in the hydrophobic matrix. The first drug and the second drug may also be a combination of drugs. One of skill in the art can envision various useful drug/matrix combinations including, but not limited to the same drug in both matrices, different drugs in each matrix and combinations of drugs in the matrices.

The drug delivery system as described herein may be provided in a kit comprising the hydrophilic matrix, the hydrophobic matrix, and a hydrating agent, such as phosphate buffered saline. The clinician can then mix the hydrophilic matrix with the hydrophobic matrix, hydrate the mixture and apply the drug delivery system to an affected site.

This drug delivery system is an ideal tool for the release of pain medication. In some situations, for example in acute post-surgical pain, where pain is most intense in the initial period post surgery it is useful to have a large spike of pain medication delivered initially and small amount thereafter as the pain intensity tapers off. However, in other situations, perhaps in the treatment of inflammation, it might be advantageous to have a more steady release from the beginning to the end. In this system, the drug release mechanisms are separated by the distribution of the drug between two different polymer matrices, one hydrophobic and one hydrophilic. The hydrophilic matrix releases its drug content by diffusion in a relatively immediate manner, e.g., within about one day. The hydrophobic matrix undergoes significant degradation immediately when exposed to physiological conditions and the drug is released over a longer period of time, e.g., within about one week. The amount of drug released more immediately and the amount of drug released subsequently can be adjusted or "tuned in" as desired by the clinician or formulator by varying the ratio of hydrophilic matrix to hydrophobic matrix.

Those skilled in the art will understand and appreciate how to adjust the respective quantities of hydrophilic and hydrophobic matrices in the systems of the present invention. The amounts will vary with several parameters including chemical structure, molecular weight, bulk density, drug concentration, drug characteristics, desired release rates, etc. A sufficient amount of the hydrophilic matrix having the first drug will be included to provide a therapeutically effective short term immediate release. A sufficient amount of the hydrophobic matrix have the second drug will be included to provide a therapeutically effective extended release. Varying the amounts of the matrices, along with the drugs, provides for a tunable system.

The following examples are illustrative of the principles and practice of the present invention although not limited thereto.

### EXAMPLES

### Example:

### Hydrophilic matrix preparation:

Hydrophilic matrices in the form of pellets were prepared containing 25% (w/w) bupivacaine HCl and 75% sodium hyaluronate. 1.25 grams of Bupivacaine HCl (minimum 99%, Sigma, St. Louis, MO) were weighed into a weighing boat. 3.75 grams of sodium hyaluronate Pharma 80 (Novamatrix/FMC Biopolymers, Philadelphia PA) were also weighed into a weighing boat. The powders were transferred to a Caleva full size mixing bowl. The mixing bowl was fixed onto the Caleva Mixer Torque Rheometer 2 (MTR 2 system) (Caleva Process Solutions, Shuminster Newton, Dorset, U.K.). The attached pair of horizontal mixing paddles mixed the powder at 50 rpm. At 60 second intervals, 1 millilter of a 50/50 ethanol/water mixture was added to the powder mixture as a wetting agent. A total of 6 milliliters was added to the powder mixture.

After mixing was complete the mixing bowl was removed from MTR 2 system and an unjacketed single screw type extruder attachment was affixed to the drive on the MTR 2 system. The Consistency Test software program (mixing time setting was 180 seconds and speed was 50 rpm and 30 seconds for logging time) was used to operate the screw type attachment. Small pieces of wet granulate were manually placed into feed inlet of screw type attachment. Granulate passed through the screw attachment and subsequently through a vertically oriented extrusion disc before emerging from extruder. Disc contained holes 2.0 millimeters in diameter. The extrudate was collected and cut into pellets 2.0 millimeters in length. The pellets were dried in a vacuum oven at room temperature overnight.

### Hydrophobic matrix preparation:

A compounder/extruder (MicroCompounder, DACA Intruments, Santa Barbara, CA) was set to 75°C and allowed to preheat for 30 minutes prior to use. 1.5 grams of Bupivacaine HCl(minimum 99%, Sigma, St. Louis, MO) were weighed out into a weighing boat. 4.5 grams of 50/50 poly(lactic acid-co-glycolic acid) (50/50 PLGA, Lakeshore Biomaterials, Birmingham, Alabama) were also weighed out. A small amount of polymer was saved while the rest of the polymer and drug were mixed together prior to feeding into the compounder. The mixture was fed into the compounder followed by the addition of the remaining polymer and compounding continued for 10 minutes until thoroughly mixed. The compounder mixing speed was 100 rpm. The final load in compounder was 2600 - 3200 Newtons. Subsequently, the mixture was allowed to extrude until the load in the system was lower than 200 Newtons. The first inch of the resin was discarded and the remainder of the extrudate was cut into pellets that were 2 mm in length and 2 mm in diameter.

Hydrophobic matrices in the form of pellets were prepared containing 25% (w/w) bupivacaine HCl and 75% PLGA. Three different PLGA matrices were prepared using PLGA IIA, a 50/50 PLGA having an inherent viscosity of 0.15 deciliter/gram, and PLGA IA, a 50/50 PLGA having an inherent viscosity of 0.1 deciliter/gram. Both PLGA IA and PLGA IIA were obtained from Lakeshore Biomaterials (Birmingham, Alabama). The first hydrophobic matrix was prepared using PLGA IIA alone. In the second matrix, a 1:1 blend of PLGA IIA and PLGA IA was used. The third matrix was a 3:1 blend of PLGA IIA and PLGA IA.

### Preparation of Bupivacaine HCL drug delivery system and controlled release analysis:

Each of the three PLGA pellets was combined with sodium hyaluronate pellets, prepared as described above, to form the drug delivery system. The ratio of PLGA pellets to sodium hyaluronate pellets was 3:1. Hansen Method I dissolution baskets containing 1 gram of the drug delivery system were attached to a Hanson SR8-PLUS dissolution test system (Hanson Research Corp. Chatsworth, CA) and lowered into respective 150 milliliter dissolution vessels and capped. Dissolution media consisted of 70% (v/v) phosphate buffer saline (PBS) with 0.01% (w/v) bovine serum albumin (BSA) / 30% (v/v) ethanol. The temperature of the dissolution apparatus was maintained at 37°C and the baskets were stirred at the rate of 25 rpm. Media samples (1 milliliter) were manually taken daily with a pipet and assayed for drug content using HPLC.

The media samples were analyzed for bupivacaine using a C-18 reverse phase column that was heated to 40°C. The gradient mobile phase began with 98% water (containing 0.01 % tri-fluoroacetic acid in water) and 2% acetonitrile and within 5 minutes changed to 100% acetonitrile, and subsequently returned to initial conditions within 2 minutes. The flow rate was 0.7 millilters/minute and the detection wavelength was 254 nanometers. Retention time for bupivacaine is 4.3 minutes.

The release results of bupivacaine HCl from the three different drug delivery systems are summarized in Table 1. Within the first day, about 30% of the drug in the three different drug delivery systems was released. All three drug delivery systems had a similar drug release in the first day since the ratio of hydrophilic to hydrophobic pellets was the same for each drug delivery system. The subsequent rate of release is what distinguished the samples. The release rate was fastest in the drug delivery system that contained PLGA pellets composed equally of PLGA IIA and IA, followed by the drug delivery system containing 3:1 PLGA IIA to IA pellets, and the drug delivery system containing the PLGA IIA pellets showed the slowest release. Therefore, the drug delivery system release rate is tunable by varying the inherent viscosity and thereby, the molecular weight of the hydrophobic polymer. The molecular weight of the polymer affects the rate of degradation of the hydrophobic matrix and therefore controls the release rate from the hydrophobic matrix.

**Table 1: Percent (%) Release of bupivacaine HCL from drug delivery systems containing 3:1 blend of PLGA pellets to sodium hyaluronatepellets**

| Time (days) | Drug delivery system containing PLGA IIA pellets | Drug delivery system containing 3:1 PLGA IIA:IA pellets | Drug delivery system containing 1:1 PLGA IIA:IA pellets |
|---|---|---|---|
| 1 | 31.4 | 26.68 | 29.24 |
| 2 | 34.85 | 36.23 | 66.65 |
| 3 | 41.72 | 77.36 | 92.61 |
| 4 | 69.89 | 92.5 | 97.72 |
| 5 | 89.5 | -- | -- |
| 6 | -- | 100 | 100.32 |
| 7 | 100 | -- | -- |

### Example 2

### Bupivacaine HCL drug delivery system preparation and controlled release analysis:

Drug delivery systems were prepared containing the sodium hyaluronate pellets (hydrophilic matrix) and the PLGA IIA pellets (hydrophobic matrix) prepared in Example 1. Three drug delivery systems were prepared using 75%, 50%, and 25% PLGA IIA pellets combined with the sodium hyaluronate pellets. These three drug delivery systems were tested along with PLGA IIA pellets alone for drug release using the dissolution method described in Example 1.

The drug release results are shown in Table 2. The PLGA IIA pellets alone had a burst of about 10% with the remaining 90% released over the week. The drug release in the first day increased with the increasing amount of sodium hyaluronate pellets. The remaining drug was released over the week upon degradation of the PLGA IIA pellets. These results demonstrate that the ratio of hydrophilic matrix to hydrophobic matrix can be used to tune the initial burst of drug, or amount of drug that is released within the first day.

**Table 2: Percent (%) Release of Bupivacaine HCl as a function of weight percent of PLGA IIA pellets to sodium hyaluronate pellets**

| Time (days) | 100% PLGA IIA pellets | 75% PLGA IIA pellets | 50% PLGA IIA pellets | 25% PLGA IIA pellets |
|---|---|---|---|---|
| 0 | 0 | 0 | 0 | 0 |
| 1 | 14.48 | 33.06 | 48.96 | 68.88 |
| 2 | 23.38 | 36.69 | 54.26 | 77.46 |
| 3 | 42.54 | 43.96 | 58.27 | 83.08 |
| 4 | 103.95 | 73.60 | 69.39 | 93.32 |
| 5 | -- | 94.57 | 78.3 | 98.32 |
| 7 | -- | 105.67 | 100.26 | 103.20 |

### Example 3

### Preparation of Ibuprofen drug delivery system and controlled release analysis:

Hydrophobic matrices and the hydrophilic matrix was prepared according to the methods of Example 1 by substituting Bupivacaine HCl with ibuprofen to test whether the same tunable drug release can be achieved with an NSAID, a different class of drug. Sodium hyaluronate pellets containing 25% (w/w) ibuprofen were made as described above. PLGA IIA pellets containing 25% (w/w) ibuprofen were prepared also as described above. The two types of pellets were combined together, 50% and 75% PLGA IIA pellets with sodium hyaluronate pellets.

The release rate of the ibuprofen from the two different drug delivery systems was compared using dissolution methods described in Example 1. The release results are summarized in Table 3. Ibuprofen was released in a similar fashion to bupivacaine HCl. An increased burst was observed with increased amount of sodium hyaluronate pellets followed by controlled release over the remaining week.

**Table 3:Percent (%) Release of ibuprofen from sodium hyaluronate and PLGA IIA pellet drug delivery systems**

| Time (days) | Percent release of ibuprofen from 50% PLGA IIA | Percent release of ibuprofen from 75% PLGA IIA |
|---|---|---|
| 0 | 0 | 0 |
| 1 | 26.7 | 19.7 |
| 2 | 54.5 | 34.3 |
| 3 | 60.6 | 42.2 |
| 4 | 77.8 | 55.3 |
| 8 | 100 | 100 |

### Example 4

### Clinical use of the drug delivery system for pain management in arthroscopic knee surgery:

A patient is evaluated for anesthesia. Arthroscopy can be performed under local, regional, or general anesthesia. The anesthesiologist determines which anesthesia is best for the patient, local conventional anesthesia in this case. Local anesthesia is administered to the patient's knee joint. The orthopaedic surgeon makes a few small incisions in the knee. A sterile solution is used to fill the knee joint and rinse away any cloudy fluid, providing a clear view of the knee. The surgeon then inserts an arthroscope to properly diagnose the source of the problem which includes torn meniscal cartilage, ruputured ancterior cruciate ligament, damaged articular cartilage, loose fragments of bone and/or cartilage, and inflamed synovial tissue. A remote TV image is used to guide the arthroscope. The surgeon then uses the appropriate conventional surgical instruments (e.g., scissors, clamps, motorized shavers, drills or lasers) and conventional implants (e.g., suture anchors, grafts, screws, sutures, tissue approximation devices) through another small incision to repair the problem using conventional surgical procedures and techniques. A drug delivery system of the present invention is prepared for administration to the patient. The ratio of hydrophilic matrix to hydrophobic matrix is chosen for the desired pain medication release profile. The matrices are mixed together and hydrated with phosphate buffered saline. The drug delivery system is then delivered to the knee. Upon completion of the surgery, the arthroscope and surgical instruments will be removed and the incisions will be closed with sutures and covered with a bandage in a conventional manner. The drug delivery system delivers pain medication to the patient during the post-surgery period.

### Example 5

### Clinical use of the drug delivery system in pain management for bone-tendon-bone reconstructive surgery:

A patient is prepared for conventional bone-tendon-bone (BTB) graft anterior cruciate ligament (ACL) reconstructive surgery in a conventional manner. Initially, a midline incision is made from the middle of the patella to the tibial tubercle. The incision depth extends just through the paratenon of the patellar tendon. The paratenon is then reflected to expose the patellar tendon. A double-bladed knife is used to make two parallel incisions through the patellar tendon, 10 mm apart. The incisions begin at the midpoint of the patella and extend distally to a point just medial to the tibial tubercle, such that the lengths of patellar and tibial bone underneath the incision are approximately 25 mm. A sagittal saw is used to remove the bone plugs along with the section of attached patellar tendon. In this manner, approximately the middle third section of the patellar tendon is harvested, with the patellar bone block on one end and the tibial bone block on the other opposed end. The thickness of the bone plugs is typically approximately 10 mm, and results in a patellar and tibial bone defect volumes of approximately 2-3 cubic centimeters. Following ACL reconstruction using BTB autograft, the patellar bone graft site is filled with bone void filler. The drug delivery system of the present invention is then prepared. The ratio of hydrophilic matrix to hydrophobic matrix is chosen for the desired pain medication release profile. The matrices are mixed together and hydrated with phosphate buffered saline. The drug delivery system is then delivered to the knee. After the patellar void is filled, the paratenon is reapproximated to cover the defect. If the paratenon is not intact, the surgical site is closed immediately after defect filling.

The novel drug delivery devices and methods of treatment have many advantages, including the ability to control via tuning drug delivery to the patient.

While the present invention has been particularly shown and described with reference to detailed embodiments thereof, it is understood that the invention is not limited to the embodiments specifically disclosed and exemplified herein. Numerous changes and modifications may be made, and such changes and modifications may be made without departing from the scope and spirit of the invention as set forth in the appended claims.

## Claims

1. A self-gelling, tunable drug delivery system comprising:
a hydrophilic matrix, which is comprised of a hydrophilic polymer that swells upon contact with a hydrating agent thereby creating a hydrogel, and a first drug, wherein said hydrophilic matrix is in the form of pellets, ribbons, films, tapes, strips, tablets or granules, and
a hydrophobic matrix, which is comprised of a hydrophobic polymer and a second drug wherein said hydrophobic matrix is in the form of pellets, ribbons, films, tapes, strips, or tablets
wherein the hydrophilic and hydrophobic matrices may be combined, hydrated and then placed in situ, or combined, placed in situ, and then hydrated.

2. The system of claim 1, wherein the hydrophilic polymer is selected from the group consisting of hydroxyethylcellulose, hydroxypropylmethylcellulose, hydroxymethylcellulose, hydroxypropylcellulose, carboxymethylcellulose, hyaluronic acids, salts of hyaluronic acid, sodium hyaluronate, alginates, polyvinylpyrrolidone, polyethylene oxide, polysccarrides, chitins, chitosan, gelatin, polyacrylic acid, derivatives of polyacrylic acid, gums, and polymers derived from starch.

3. The system of claim 1, wherein the hydrophilic polymer comprises high molecular weight sodium hyaluronate.

4. The system of claim 1, wherein the hydrophobic polymer comprises an aliphatic polyester selected from the group consisting of lactide, glycolide, epsilon-caprolactone, p-dioxanone, and trimethylene carbonate and copolymers and terpolymers thereof.

5. The system of claim 4, wherein the aliphatic polyester has a melt processing temperature less than 100°C.

6. The system of claim 4, wherein the aliphatic polyester comprises 50/50 mol/mol percent poly(lactic acid-co-glycolic acid).

7. The system of claim 1, wherein the first drug and the second drug are selected from the group consisting of anti-infectives, analgesics, anesthetics, immunosupressives, steroids, statins, alpha-2-agonists, VR1-agonists, proton pump inhibitors, collagen peptides, parathyroid hormone, bone morphogenic proteins, p38 kinase inhibitors and combinations thereof.

8. The system of claim 1, wherein the first drug and the second drug are selected from the group consisting of ibuprofen, oxycodone, morphine, fentanyl, hydrocodone, naproxyphene, codeine, acetaminophen with codeine, acetaminophen, benzocaine, lidocaine, procaine, bupivacaine, ropivacaine, mepivacaine, chloroprocaine, tetracaine, cocaine, etidocaine, prilocaine, procaine, clonidine, xylazine, medetomidine, dexmedetomidine, VR1 antagonists and combinations thereof.

9. The system of claim 1 wherein the first drug and the second drug are bupivacaine or lidocaine.

10. The system of claim 1 wherein the first drug and the second drug are the same.

11. The system of claim 1, wherein the first drug is different than the second drug.

12. A system as defined in any of claims 1 to 11 for use in a method for treating pain comprising the steps of mixing the hydrophilic matrix with the hydrophobic matrix, hydrating the mixture to form a hydrogel, and applying the hydrogel locally to an affected area, wherein the first drug and the second drug may be the same or different and are selected from analgesics.

13. The system of claim 1 in the form of a kit comprising the hydrophilic matrix, the hydrophobic matrix, and a hydrating agent.

## Patentansprüche

1. Selbstgelierendes, abstimmbares Arzneimittelzuführungssystem, umfassend:
eine hydrophile Matrix, bestehend aus einem hydrophilen Polymer, das nach Kontakt mit einem Hydratisierungsmittel quillt und dadurch ein Hydrogel bildet, und einem ersten Arzneimittel, wobei die hydrophile Matrix in Form von Pellets, Bändern, Filmen, Klebebändern, Streifen, Tabletten oder Granula vorliegt, und
eine hydrophobe Matrix, die aus einem hydrophoben Polymer und einem zweiten Arzneimittel besteht, wobei die hydrophobe Matrix in Form von Pellets, Bändern, Filmen, Klebebändern, Streifen oder Tabletten vorliegt, wobei die hydrophile und die hydrophobe Matrix vereinigt, hydratisiert und dann in situ platziert oder vereinigt, in situ platziert und hydratisiert werden können.

2. System nach Anspruch 1, wobei das hydrophile Polymer aus der Gruppe ausgewählt ist, die aus Hydroxyethylcellulose, Hydroxypropylmethylcellulose, Hydroxymethylcellulose, Hydroxypropylcellulose, Carboxymethylcellulose, Hyaluronsäuren, Salzen von Hyaluronsäure, Natriumhyaluronat, Alginaten, Polyvinylpyrrolidon, Polyethylenoxid, Polysccarriden, Chitinen, Chitosan, Gelatine, Polyacrylsäure, Derivaten von Polyacrylsäure, Gummen und von Stärke stammenden Polymeren besteht.

3. System nach Anspruch 1, wobei das hydrophile Polymer hochmolekulares Natriumhyaluronat umfasst.

4. System nach Anspruch 1, wobei das hydrophobe Polymer einen aliphatischen Polyester umfasst, der aus der aus Lactid, Glykolid, epsilon-Caprolacton, p-Dioxanon und Trimethylencarbonat sowie Copolymeren und Terpolymeren davon bestehenden Gruppe ausgewählt ist.

5. System nach Anspruch 4, wobei der aliphatische Polyester eine Schmelzverarbeitungstemperatur von weniger als 100°C hat.

6. System nach Anspruch 4, wobei der aliphatische Polyester 50/50 mol/mol-Prozent an Poly(Milchsäure-Co-Glykolsäure) umfasst.

7. System nach Anspruch 1, wobei das erste Arzneimittel und das zweite Arzneimittel aus der Gruppe ausgewählt sind, die aus Antiinfektiva, Analgetika, Anästhetika, Immunsuppressiva, Steroiden, Statinen, alpha-2-Agonisten, VR1-Agonisten, Protonenpumpenhemmern, Kollagenpeptiden, Parathormon, knochenmorphogenen Proteinen, p38-Kinaseinhibitoren und Kombinationen davon besteht.

8. System nach Anspruch 1, wobei das erste Arzneimittel und das zweite Arzneimittel aus der Gruppe ausgewählt sind, die aus Ibuprofen, Oxycodon, Morphin, Fentanyl, Hydrocodon, Naproxyphen, Codein, Acetaminophen mit Codein, Acetaminophen, Benzocain, Lidocain, Procain, Bupivacain, Ropivacain, Mepivacain, Chlorprocain, Tetracain, Kokain, Etidocain, Prilocain, Procain, Clonidin, Xylazin, Medetomidin, Dexmedetomidin, VR1-Antagonisten und Kombinationen davon besteht.

9. System nach Anspruch 1, wobei das erste Arzneimittel und das zweite Arzneimittel Bupivacain oder Lidocain sind.

10. System nach Anspruch 1, wobei das erste Arzneimittel und das zweite Arzneimittel gleich sind.

11. System nach Anspruch 1, wobei das erste Arzneimittel von dem zweiten Arzneimittel verschieden ist.

12. System nach einem der Ansprüche 1 bis 11 für die Verwendung bei einem Verfahren zur Behandlung von Schmerz, umfassend die Schritte Mischen der hydrophilen Matrix mit der hydrophoben Matrix, Hydratisieren des Gemischs unter Bildung eines Hydrogels und Aufbringen des Hydrogels lokal auf einen betroffenen Bereich, wobei das erste Arzneimittel und das zweite Arzneimittel gleich oder verschieden sein können und aus Analgetika ausgewählt sind.

13. System nach Anspruch 1 in Form eines Kits, das die hydrophile Matrix, die hydrophobe Matrix und ein Hydratisierungsmittel umfasst.

## Revendications

1. Système d'administration de médicament accordable auto-gélifiant, comprenant :
une matrice hydrophile, qui comprend un polymère hydrophile qui gonfle au contact d'un agent hydratant, créant ainsi un hydrogel, et un premier médicament, ladite matrice hydrophile étant sous la forme de pastilles, de rubans, de films, de bandes, de bandelettes, de comprimés ou de granules, et
une matrice hydrophobe, qui comprend un polymère hydrophobe et un second médicament, ladite matrice hydrophobe étant sous la forme de pastilles, de rubans, de films, de bandes, de bandelettes ou de comprimés,
les matrices hydrophile et hydrophobe pouvant être combinées, hydratées, puis placées in situ, ou combinées, placées in situ, puis hydratées.

2. Système selon la revendication 1, dans lequel le polymère hydrophile est choisi dans le groupe constitué par l'hydroxyéthylcellulose, l'hydroxypropylméthylcellulose, l'hydroxyméthylcellulose, l'hydroxypropylcellulose, la carboxyméthylcellulose, les acides hyaluroniques, les sels d'acide hyaluronique, l'hyaluronate de sodium, les alginates, la polyvinylpyrrolidone, l'oxyde de polyéthylène, les polysaccharides, les chitines, le chitosan, la gélatine, l'acide polyacrylique, les dérivés de l'acide polyacrylique, les gommes et les polymères dérivés d'amidon.

3. Système selon la revendication 1, dans lequel le polymère hydrophile comprend du hyaluronate de sodium de poids moléculaire élevé.

4. Système selon la revendication 1, dans lequel le polymère hydrophobe comprend un polyester aliphatique choisi dans le groupe constitué par le lactide, le glycolide, l'epsilon-caprolactone, la p-dioxanone, et le carbonate de triméthylène et leurs copolymères et terpolymères.

5. Système selon la revendication 4, dans lequel le polyester aliphatique a une température de traitement à l'état fondu inférieure à 100 °C.

6. Système selon la revendication 4, dans lequel le polyester aliphatique comprend 50/50 pour cent mole/mole de poly(acide lactique-co-acide glycolique).

7. Système selon la revendication 1, dans lequel le premier médicament et le second médicament sont choisis dans le groupe constitué par les anti-infectieux, les analgésiques, les anesthétiques, les immunosuppresseurs, les stéroïdes, les statines, les agonistes alpha-2, les agonistes de VR1, les inhibiteurs de la pompe à protons, les peptides de collagène, l'hormone parathyroïdienne, les protéines morphogéniques des os, les inhibiteurs de kinase p38 et leurs combinaisons.

8. Système selon la revendication 1, dans lequel le premier médicament et le second médicament sont choisis dans le groupe constitué par l'ibuprofène, l'oxycodone, la morphine, le fentanyl, l'hydrocodone, le naproxyphène, la codéine, l'acétaminophène avec la codéine, l'acétaminophène, la benzocaïne, la lidocaïne, la procaïne, la bupivacaïne, la ropivacaïne, la mépivacaïne, la chloroprocaïne, la tétracaïne, la cocaïne, l'étidocaïne, la prilocaïne, la procaïne, la clonidine, la xylazine, la médétomidine, la dexmédétomidine, les antagonistes de VR1 et leurs combinaisons.

9. Système selon la revendication 1, dans lequel le premier médicament et le second médicament sont la bupivacaïne ou la lidocaïne.

10. Système selon la revendication 1, dans lequel le premier médicament et le second médicament sont identiques.

11. Système selon la revendication 1, dans lequel le premier médicament est différent du second médicament.

12. Système tel que défini dans l'une quelconque des revendications 1 à 11, destiné à une utilisation dans une méthode de traitement de la douleur, comprenant les étapes de mélange de la matrice hydrophile avec la matrice hydrophobe, d'hydratation du mélange pour former un hydrogel et d'application de l'hydrogel localement sur une zone affectée, le premier et le second médicament pouvant être identiques ou différents, et étant choisis parmi les analgésiques.

13. Système selon la revendication 1, sous la forme d'un kit comprenant la matrice hydrophile, la matrice hydrophobe et un agent hydratant.
